# EUROPEAN PATENT APPLICATION

(11) **EP 4 681 672 A1**
(43) Date of publication of application: **21.01.2026**
(21) Application number: 24188921.1
(22) Date of filing: 16.07.2024
(51) Int. Cl.: A61B 18/22

(54) **A PROTECTIVE ASSEMBLY FOR USE IN A THERAPEUTIC LASER SYSTEM**

(71) Applicant: Ferton Holding S.A., 2800 Delémont (CH)
(72) Inventor: MENAESSE, Audrey, 1020 Renens (CH)
(74) Representative: Osha BWB

(57) **Abstract**

According to a first aspect, the present disclosure relates to a protective assembly for use in a therapeutic laser system, the therapeutic laser system configured to direct a therapeutic laser beam to a treatment area, the therapeutic laser system comprising a laser unit and an optical free space area disposed between the laser unit and the treatment area, the laser unit being configured to output the therapeutic laser beam having a first optical path through said optical free space area to the treatment area and to output an aiming beam having a second optical path through said optical free space area to the treatment area, the protective assembly comprising: a protective window configured to be disposed between the optical free space area and the treatment area so as to intersect the first optical path and the second optical path, the protective window at least partially transparent to the therapeutic laser beam and to the aiming beam; an optical sensor configured to measure an optical parameter of light from the aiming beam or the therapeutic laser beam scattered at the protective window; and a control unit configured to determine if the measured optical parameter is within a predetermined range.

## Description

### TECHNICAL FIELD OF THE INVENTION

The present description relates to protective assemblies for use in laser systems. Further, the present description relates to laser systems incorporating protective assemblies and methods of using such protective assemblies.

### BACKGROUND OF THE INVENTION

Therapeutic laser systems, such as those used in the field of urology, provide a therapeutic laser beam from a laser to a tissue bed, or other work area, via an optical fiber. Therapeutic laser systems can include device safety elements comprised in an optical free space area, or safety optical bench, positioned between the laser and the optical fiber.

Device safety elements may be used to control the power delivered by the optical fiber, for example, during periods of instability of the laser (REF [1]), or when the optical fiber is damaged (REF [2]). Device safety elements comprise a number of optical elements, such as mirrors or lenses. Device safety elements may comprise optical elements arranged to steer the therapeutic beam, or part thereof, to a beam dump (REF [3]). In addition to the device safety elements, the optical free space area comprises a lens to focus the therapeutic beam, following modification or processing by the device safety elements, onto the proximal end of the optical fiber (REF [3]).

If the therapeutic beam is not perfectly aligned with the core of the optical fiber, the optical fiber can blast or catch on fire, producing shrapnel which can damage the optical elements inside the optical free space area. In addition, there can be a fire risk and/or a risk of damage to the optical elements with the optical free space area if any dust or humidity droplets are present at the surface of an optical element of the optical bench.

Furthermore, the optical fiber may become damaged, such as breaks in the core, or contaminated by blood or materials on the distal end of the optical fiber at the tissue bed or work area. Damage or contamination of the optical fiber can cause thermal heating of the optical fiber due to trapped laser energy, which can then result in melting or vaporization of a part of the optical fiber. The melting or vaporization of the optical fiber can cause damage to the components of the optical free space area.

It is known in the art to use mechanical protective windows (also referred to as blast shields) between the optical free space area and the optical fiber to protect the optical elements in the optical free space area (REF [4]). However, any damage to or contamination of the protective window will affect the therapeutic beam being delivered to the work site. Therefore, proper maintenance of the protective window is required.

### SUMMARY

In what follows, the term "comprise" is synonym of (means the same as) "include" and "contains", is inclusive and open, and does not exclude other non-recited elements. Moreover, in the present disclosure, when referring to a numerical value, the terms "about" and "substantially" are synonyms of (mean the same as) a range comprised between 80% and 120%, preferably between 90% and 110%, of the numerical value.

According to a first aspect, the present description relates to a protective assembly for use in a therapeutic laser system, the therapeutic laser system configured to direct a therapeutic laser beam to a treatment area, the therapeutic laser system comprising a laser unit and an optical free space area disposed between the laser unit and the treatment area, the laser unit being configured to output the therapeutic laser beam having a first optical path through said optical free space area to the treatment area and to output an aiming beam having a second optical path through said optical free space area to the treatment area, the protective assembly comprising:
a protective window configured to be disposed between the optical free space area and the treatment area so as to intersect the first optical path and the second optical path, the protective window at least partially transparent to the therapeutic laser beam and to the aiming beam;
an optical sensor configured to measure an optical parameter of light from the aiming beam or the therapeutic laser beam scattered at the protective window; and
a control unit configured to determine if the measured optical parameter is within a predetermined range.

According to an embodiment, partially transparent indicates that the protective window, under normal working conditions (i.e. not damaged or contaminated), is transparent to at least 75% of the therapeutic laser beam, and is transparent to at least 75% of the aiming beam.

The protective assembly provides an improved means of protecting the optical free space area of the therapeutic laser system. The protective assembly enables a condition of the protective window to be monitored, by measuring the optical parameter of light scattered at the protective window and determining if the optical parameter is within a predetermined range.

According to an embodiment, the protective window comprises a first window face and a second window face, where the first window face and the second window face are planar and parallel.

According to an embodiment, the optical sensor is configured to measure the optical parameter of the light from the aiming beam or the therapeutic laser beam that is scattered by a defect in the protective window or by debris that is deposited on a face of the protective window.

According to an embodiment, the optical sensor comprises one of a photodiode, a phototransistor, a photoresistor, an array of photodiodes, phototransistors, or phototransistors, or a camera.

According to an embodiment, the optical sensor further comprises an optical lens configured to direct light on an active area of the optical sensor.

According to an embodiment, the protective window is at least partially transparent in a range of about 280 nm to about 100 µm. This enables the protective window to be used with both visible and infrared wavelengths.

According to an embodiment, the control unit is further configured to provide an indication to a user of the therapeutic laser system to inspect the protective window. By providing the indication to the user, damaged or contaminated protective windows can be inspected and replaced. By continuously monitoring the protective window and providing the indication to the user, this reduces the need for a user to regularly open the therapeutic laser system to visually check the condition of the protective window.

According to an embodiment, the optical sensor is positioned relative to the protective window so that a cone of acceptance of the optical sensor encompasses at least part of the protective window, wherein the cone of acceptance extends at least 1mm from a first window face of the protective window in the direction of the optical free space area and/or extends at least 1mm from a second window face of the protective window in a direction of the treatment area. The cone of acceptance defines a maximum angle with respect to an optical axis of the optical sensor at which the scattered light will be incident on an active area of the optical sensor and is also referred to as the angular optical field. According to an embodiment, the optical axis of the optical sensor is an axis that passes through a center of an active area of the optical sensor, where the optical axis is perpendicular to a plane comprising the active area. When the optical sensor further comprises an optical lens to direct light on said active area, the angular optical field is determined in a known way based on a focal distance of said optical lens and a dimension of said active area. This will ensure that at least a portion light scattered from debris on the first window face or second window face can be detected by the optical sensor.

According to an embodiment, the optical sensor is positioned relative to the protective window so as to measure the optical parameter of the light scattered in a direction forming an angle less than 20° with a first window face of the protective window and/or forming an angle less than 20° with a second window face of the protective window. Such an arrangement ensures that light scattered substantially perpendicular to an optical axis of the therapeutic beam or aiming beam at the protective window is captured by the optical sensor, along with light scattered within 20 ° of the first window face and the second widow face.

According to an embodiment, the protective window comprises an anti-reflection coating. This reduces reflection from the protective window during normal use (i.e. when not damaged or contaminated). This reduces baseline scattering that may affect the measurement of the scattering due to the damage or contamination of the protective window.

According to an embodiment, the optical parameter is an optical intensity, a polarisation or an optical power.

According to an embodiment, the protective assembly further comprises a removable mounting, the removable mounting comprising a window support member configured to support the protective window; and an attachment member mechanically coupled to the window support member and configured to releasably attach the protective assembly to a housing of the therapeutic laser system. The removable mounting allows for the ease of replacement of the protective window in the therapeutic laser system.

According to a second aspect, the present description relates to a therapeutic laser system configured to direct a therapeutic laser beam to a treatment area, the therapeutic laser system (100) comprising: a laser unit configured to output the therapeutic laser beam having a first optical path through an optical free space area to the treatment area and to output an aiming beam having a second optical path through the optical free space area to the treatment area; the optical free space area (114) disposed between the laser unit and the treatment area; and a protective assembly (102) according to the first aspect. A therapeutic laser system comprising a protective assembly that monitors a protective window improves the safety related to using the therapeutic laser system as any damage or contamination of the protective window may be identified effectively.

According to an embodiment, the laser unit comprises a first light source configured to output the therapeutic laser beam), the first light source being one of a Thulium:YAG laser, a Holium:YAG laser, a KTP (Potassium titanyl phosphate):YAG laser, Nd (Neodymium):YAG laser, a carbon dioxide (CO2) laser or a diode laser.

According to an embodiment, the laser unit comprises a second light source configured to output the aiming beam, the second light source being one of a laser diode, a laser or a light emitting diode configured to emit light in the range of 280 nm to 700 nm.

According to an embodiment, the optical free space area comprises a lens configured to direct the therapeutic laser beam and the aiming beam toward a proximal end of an optical fiber, the optical fiber disposed between the protective window and the treatment area, and wherein the protective window of the protective assembly is disposed between the lens and the proximal end of the optical fiber.

According to an embodiment, the therapeutic laser system further comprises a fiber coupler configured to couple to the optical fiber; and, optionally, further comprises the optical fiber.

According to a third aspect, the present description relates to a method of monitoring a protective window of a therapeutic laser system configured to direct a therapeutic laser beam to a treatment area, the therapeutic laser system comprising a laser unit configured to output the therapeutic laser beam having a first optical path through an optical free space area to the treatment area and to output an aiming beam having a second optical path through the optical free space area to the treatment area, the laser system further comprising the optical free space area disposed between the laser unit and the treatment area, and a protective window disposed between the optical free space area and treatment area so as to intersect the first optical path and the second optical path, the protective window at least partially transparent to the therapeutic laser beam and the aiming beam, the method comprising:
measuring with an optical sensor an optical parameter of light from the aiming beam or the therapeutic laser beam scattered at the protective window; and
determining, using a control unit, if the measured optical parameter is within a predetermined range.

According to an embodiment, the method further comprises providing, by the control unit, based on the determining, an indication to a user of the therapeutic laser system to inspect the protective window.

### BRIEF DESCRIPTION OF DRAWINGS

Other advantages and features of the invention will become apparent on reading the description, illustrated by the following figures which represent:
- FIG. 1 illustrates a therapeutic laser system comprising a protective assembly, according to an embodiment.
- FIG. 2 illustrates a protective assembly, according to an embodiment.
- FIG. 3 illustrates a removable mounting configured to hold or support the protective window of the protective assembly, according to an embodiment.
- FIG. 4 outlines the steps of a method of monitoring a protective window, according to an embodiment.

### DETAILED DESCRIPTION

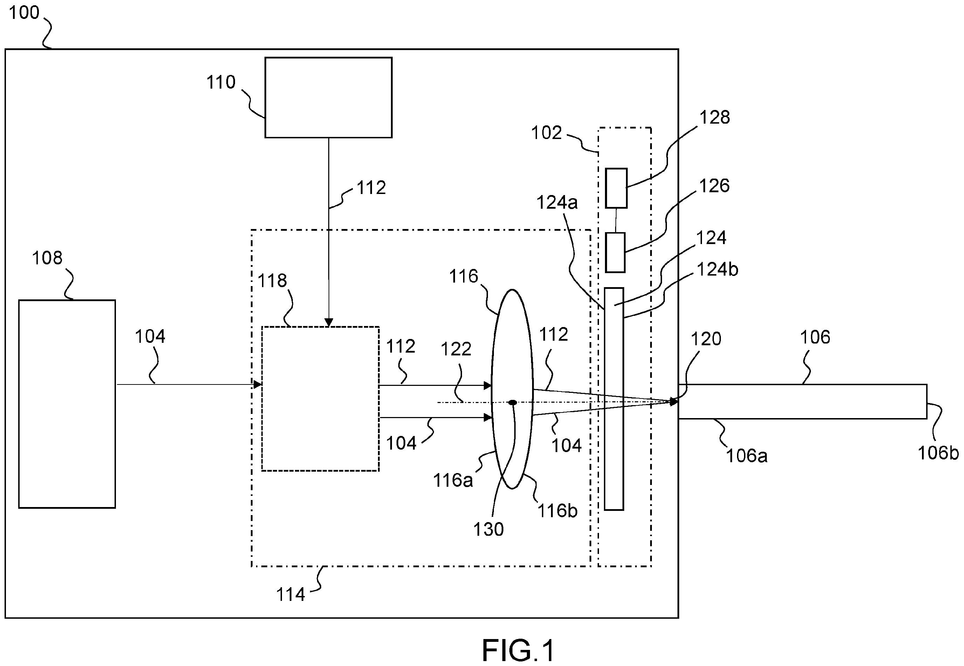

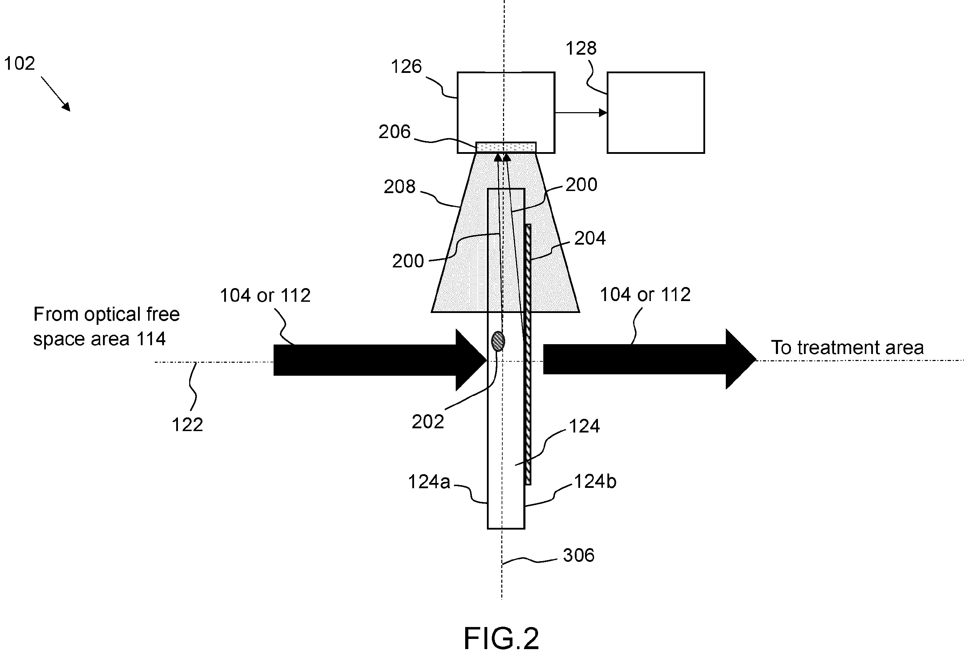

FIG. 1 illustrates a therapeutic laser system 100 comprising a protective assembly 102 according to an embodiment. According to an embodiment, the protective assembly 102 is removable from the therapeutic laser system 100 so as to allow for replacement of the protective assembly 102, as detailed further below. FIG.2 illustrates a detailed view of the protective assembly 102 for use in the therapeutic laser system 100 according to an embodiment. According to an embodiment, the therapeutic laser system 100 is for use in urology, dentistry or pain therapy.

The therapeutic laser system 100 of FIG. 1 is configured to direct a therapeutic laser beam 104 into a proximal end 106a of an optical fiber 106, so that the optical fiber 106 can deliver the therapeutic beam 104 to a treatment area (not shown), such as bodily tissue. The optical fiber 106 comprises the proximal end 106a and a distal end 106b. The proximal end 106a refers to an end of the optical fiber 106 that is closest or nearest to the therapeutic laser system 100 and the protective assembly 102. The distal end 106b refers to the end of the optical fiber 106 that is furthest away from the therapeutic laser system 100 and the protective assembly 102, and closest to the treatment area.

According to an embodiment, the optical fiber 106 is not comprised in the therapeutic laser system 100. The optical fiber 106 may be a fiber that is changed for different applications or following damage to the optical fiber 106, changed as part of a routine protocol, or changed for use with different patients. The optical fiber 106 may be a single-mode or multi-mode fiber. The optical fiber 106 may be of various different lengths, diameters or materials. It will be clear to a person of ordinary skill in the art that the type of optical fiber 206 that the therapeutic laser system 100 is suitable for use with will depend on parameters such as the application the therapeutic laser system 100 is to be used for, and the other optical components (such as light sources and lens) used within the therapeutic laser system 100. As an example only, the type of optical fiber 106 used may be selected based on a desired optical coupling efficiency of the therapeutic laser beam 104 from the therapeutic laser system 100 into the optical fiber 106.

According to an embodiment, the therapeutic laser system 100 may be configured to direct the therapeutic laser beam 104 to the treatment site without the optical fiber 106.

Referring back to FIG.1, the therapeutic laser system 100 comprises a first light source 108 configured to emit the therapeutic laser beam 104 having a first wavelength. Herein, a beam refers to a bundle of electromagnetic waves that remain essentially concentrated around a mean axis upon free propagation. The first light source 108 is a laser. According to an embodiment, the first light source 108 is a diode laser, such as a visible diode laser, a UV diode laser, or an infrared diode laser. According to an embodiment, the first light source 108 is a Thulium fiber laser or Thulium:YAG (yttrium-aluminum-garnet) laser, and the first wavelength is in the range 1810 nm to 2100 nm. According to an embodiment the first wavelength is substantially equal to 1940 nm. According to an embodiment, the first light source 108 is a Holmium:YAG laser, and the first wavelength is substantially equal to 2100 nm. According to an embodiment, the first light source 108 is a KTP (Potassium titanyl phosphate):YAGlaser and the first wavelength is substantially equal to 532 nm. According to an embodiment, the first light source 108 is a Nd (Neodymium):YAGlaser and the first wavelength is substantially equal to 1064nm. According to an embodiment, the first light source 108 is a carbon dioxide (CO₂) laser, and the first wavelength is substantially in the range 9.3 µm to 10.6 µm.

According to an embodiment, the first wavelength of the therapeutic laser beam 104 is in the infrared range of wavelengths of light. For example, the infrared range includes wavelengths in the range of 780 nm to 100 µm.

The therapeutic laser system 100 further comprises a second light source 110 configured to emit an aiming beam 112 having a second wavelength. The function of the aiming beam is to producing a visible or non-visible spot, intended for indication of the anticipated point of impact of the therapeutic laser beam 104, as explained for example in (REF 5). Thus, the aiming beam 112 enables a user of the therapeutic laser system 100 to determine where the therapeutic laser beam 104 is being aimed on the tissue or work site.

According to an embodiment, the second light source 110 is one of a light emitting diode, a laser, or a laser diode. According to an embodiment, the second wavelength is in the visible range of light. For example, the visible range of wavelengths of light may be considered to be the range 280 nm to 700 nm. Alternatively, the aiming beam 112 may be a non-visible wavelength, with a user using a camera, goggles, or other tool to enable the aiming beam 112 to be observed. According to an embodiment, the aiming beam 112 is a non-laser beam or non-coherent light beam. According to an embodiment, the aiming beam 112 is a laser beam or coherent beam.

It will be clear to a person of ordinary skill in the art that the first light source 108 and the second light source 110 may be considered to form a laser unit, where the laser unit is configured to emit (or output) the therapeutic laser beam 104 and the aiming beam 112.

The therapeutic laser beam 104 has a first optical path (not shown) between the first light source 108 and the treatment area. The first optical path is the trajectory that the therapeutic laser beam 104 follows as it propagates from the laser unit (or the first light source 108 of the laser unit) to the treatment area. The aiming beam 112 defines a second optical path (not shown) between the laser unit (or the second light source 110 of the laser unit) and the treatment area. The second optical path is the trajectory that the aiming beam 112 follows as it propagates from the second light source 110 to the treatment area. According to an embodiment, the first optical path and the second optical path are different along at least a portion of the paths.

The therapeutic laser system 100 further comprises an optical free space area 114 comprising portions of the first optical path and the second optical path between the laser unit (first light source 108 and the second light source 110) and the treatment area. The optical free space area 114 is an area where the therapeutic laser beam 104 propagates through free space along a portion of the first optical path comprised within the optical free space area 214, and where the aiming beam 112 propagates through free space along a portion of the second optical path comprised within the optical free space area 114. Free space is air or a vacuum. The optical free space area 114 may be referred to as a safety optical bench.

According to an embodiment, the optical free space area 114 optionally comprises a number of safety elements 118. The safety elements 118 are disposed along the portion of the first optical path comprised within the optical free space area 114 and/or the portion of the second optical path comprised within the optical free space area 114. The safety elements 118 thereby interest the first optical path and/or the second optical path, so that the therapeutic laser beam 104 and/or the aiming beam 112 are incident on at least some of the safety elements.

The safety elements 118 may comprise elements such as mirrors, lens, beam dumps, and shutters. The safety elements 118 may be used to modify, measure, redirect or inhibit the therapeutic laser beam 104 so as to comply with a set of safety standards. The safety elements 118 may also be used to modify, measure, redirect or inhibit the aiming beam 112. According to an embodiment, the safety elements 118 direct the aiming beam 112 and the therapeutic laser beam 104 towards the lens 116.

Within the optical free space area 114, a safety element of the safety elements 118 may be disposed along the first optical path, the second optical path or along both the first and second optical paths. For example, the therapeutic laser beam 104 may pass through a first number of safety elements 118, and the aiming beam 112 may be passed through a second number of safety elements 118, where the second number is different from the first number.

It will be clear to a person of ordinary skill in the art that applications may arise where no safety elements are required, for example in the case of low-powered lasers, and that the safety elements 118 are as such optional.

According to an embodiment, the optical free space area 114 comprises a lens 116 configured to direct the therapeutic laser beam 104 and the aiming beam 112 to the treatment area. In an embodiment where the therapeutic laser system (100) comprises the optical fiber 106, such as in FIG. 1, the lens 116 is configured to direct the therapeutic beam 104 and the aiming beam 112 toward the proximal end 106a of the optical fiber 106. The lens 116 of the optical free space area 114 is separated from the laser system (first light source 108 and second light source 110) and the treatment area (or the proximal end 106a of the optical fiber 106) by free space. between the laser unit and the lens 116. The lens 116 is disposed between the laser unit and the treatment area along the first optical path and along the second optical path. In an embodiment where safety elements 118 are comprised in the therapeutic laser system 100, the lens 116 is disposed between the safety elements 118 and the treatment area.

According to an embodiment, the lens 216 is a focusing lens configured to converge the therapeutic laser beam 104 and the aiming beam 112 passing through the lens 116 towards a point of convergence 120.

The lens 116 comprises a first lens surface 116a proximal to the laser unit and a second lens surface 116b distal to the laser unit. According to an embodiment, the first lens surface 116a or the second lens surface 116b is a curved surface. According to an embodiment, the lens 116 is a double convex lens (as illustrated in FIG. 1 and FIG. 2). Alternatively, the lens 116 is a plano-convex lens, where the first lens surface 116a is a convex surface and the second lens surface 116b is planar. Alternatively, the lens 116 is convex-concave (or meniscus) lens, where the first lens surface 116a is convex and the second lens surface 116b is concave. According to an embodiment, the first lens surface 116a and/or the second lens surface 116b may be planar, spherical, aspherical, or cylindrical.

The lens 116 has an optical axis 122 that joins a center of curvature of the first lens surface 116a, a center of curvature of the second lens surface 116b and an optical center 130 of the lens 116. The optical center 130 of the lens 216 is the point on the lens 216 through which a ray of light passes without deviation. A focal point of the lens 216 is a point on the optical axis 122 where light rays, parallel to the optical axis 122 converge after passing through the lens 116. The focal length of the lens 116 is the distance between the optical center 130 of the lens 116 and the focal point.

FIG. 1 and FIG. 2 illustrate an arrangement where the therapeutic laser beam 104 and the aiming beam 112 are substantially parallel to the optical axis 122 of the lens 116 and so the point of convergence 120 of the therapeutic laser beam 104 and aiming beam 112 coincides with the focal point of the lens 216.

Alternatively, the lens 116 may be arranged so that the therapeutic laser beam 104 and the aiming beam 112 are not parallel to the optical axis 122. In such an arrangement, the point of convergence 120 will lie on a focal plane, where the focal plane is a plane perpendicular to the optical axis 122 that comprises the focal point.

In FIG.1, the point of convergence 120 of the therapeutic laser beam 104 and the aiming beam 112 is shown to be located at the proximal end 106a of the optical fiber 106. In the arrangement of FIG.1, the distance between the proximal end 106a of the optical fiber 106 and the center of the lens 116 is equal to the focal length, so that the therapeutic laser beam 104 and the aiming beam 112 are focused to the focal point at the point of convergence 120 at the proximal end 106a of the optical fiber 106.

However, depending on the application and the degree of coupling efficiency of the therapeutic laser beam 104 into the optical fiber 106 required, the proximal end 106a of the optical fiber 106 could be a distance less than, or greater to the focal length from the lens 116. In coupling the therapeutic laser beam 104 and the aiming beam 112 to the optical fiber 106, the function of the lens 116 is to focus the therapeutic laser beam 104 and the aiming beam 112 so that they have a spot size (beam diameter) that is suitable for coupling into the optical fiber 106. The required beam diameter will depend on the application, the therapeutic laser beam 104 diameter emitted from the first light source 108, and the features of the optical fiber 106 such as numerical aperture (NA) and core size. Depending on the lens 116, the desired spot size or beam diameter may be achieved at a distance less than the focal length, or as the beams diverge again after the point of convergence 120 in a direction toward the optical fiber, the spot size at a distance greater than the focal length may still be of a diameter suitable for coupling to the optical fiber 106.

The protective assembly 102 comprises a protective window 124, an optical sensor 126 and a control unit 128. As illustrated in FIG. 2, the protective window 224 is disposed along the first optical path and the second optical path between the optical free space area 114 and the treatment area, so that the protective window 224 intersects the first optical path and the second optical path. The function of the protective window 124 is to form a barrier that separates the environment of the optical free space area 114, and the optical components within it, from the optical fiber 106, while allowing the therapeutic laser beam 104 and the aiming beam 112 to pass from the optical free space area 114 to the treatment area (or optical fiber 106). According to an embodiment, the protective window 124 does not substantially modify the therapeutic laser beam 104 or the aiming beam 112. According to an embodiment, the protective window 124 is not a lens or other element configured to substantially refract or reflect the therapeutic laser beam 104 or the aiming beam 112.

The protective window 124 is at least partially optically transparent to the first wavelength and the second wavelength. The protective window 124 may also be designed and fabricated to minimize reflection and absorption at the first wavelength and the second wavelength. According to an embodiment, "partially optically transparent" indicates that the protective window 124 under normal working conditions (i.e. not damaged or contaminated) is transparent to at least 75% of the therapeutic laser beam 104 at the first wavelength, and is transparent to at least 75% of the aiming beam 112 at the second wavelength. According to an embodiment, the protective window 124 under normal working conditions is substantially optically transparent to the first wavelength and the second wavelength. It will be clear to a person of ordinary skill in the art that any defects in the protective window 124 or contamination of the protective window 124 may affect the optical transparency of the protective window 124.

The protective window 124 comprises a first window face 124a proximal to the lens 116, and a second window face 124b distal to the lens 116, wherein the first window face 124a and the second window face 124b are disposed along both the first optical path and the second optical path.

In FIG. 1 and FIG.2, the first window face 124a and the second window face 124b are substantially parallel, forming a protective window 124 of substantially uniform thickness. In an embodiment, the second window face 124b is at an angle less than 5 arc seconds (or 5/3600 degrees) from the first window face 124a.

Scratch-dig is a qualitative technique for classifying level of polish for optics, where a scratch is an elongated mark on an optical surface and a dig is a round pit or mark, including a bubble, in the optical surface. A scratch-dig designation is specified by a set of two numbers such as 60-40. The first number (e.g. 60) relates to the maximum width allowance of a scratch as measured in microns. Additionally, the combined length of maximum-size scratches on the surface of the optic in question cannot exceed ¼ the diameter of the usable lens area. The second number (e.g. 40) indicates the maximum diameter allowance for a dig in the optic in hundredths of a millimeter. The allowable number of maximum size digs within the useful area of the lens is one, and the sum of the diameters of all digs cannot exceed twice the diameter of the minimum size dig number specified. According to an embodiment, a scratch-dig designation of the first window face 124a and the second window face 124b is 40-20 or 20-10. For a scratch-dig designation of 20-10, the maximum scratch width is 0.02mm and the maximum dig diameter is 0.1mm.

According to an embodiment, and as illustrated in FIG. 2, the protective window 124 is substantially perpendicular to the optical axis 122 of the lens 116.

However, it will be clear to the person of ordinary skill in the art that the first window face 124a and the second window face 124b may not be parallel, and the protective window 124 may not be of substantially uniform thickness. According to an embodiment, the first window face 124a and/or the second window face 124b may be at least partially curved. Furthermore, it will be clear to the person of ordinary skill in the art that the protective window 124 may be placed at an angle to the optical axis 122 other than 90°.

The protective window 124 may be made from any material that is substantially transparent to the first wavelength and the second wavelength, such as glass or plastic. Example materials include UV fused silica, or optical quartz such as Infrasil^{®}.

According to an embodiment, the protective window 124 may comprise an anti-reflective coating (not shown) on the first window face 124a and/or the second window face 124b. Such coatings will minimize reflection of the therapeutic laser beam 104 and the aiming beam 112 during normal operation (i.e. when the protective window 124 is not damaged or contaminated).

Referring to FIG. 2, a portion of light 200 from the therapeutic laser beam 104 and/or the aiming beam 112 may be scattered at the protective window 124. For example, the portion of light 200 may be scattered by a defect 202 within the protective window 124, where the defect 202 may have been caused by a blast of a fire arising due to the therapeutic beam 104 being not perfectly aligned with the core of the optical fiber 106. The portion of light 200 may also be scattered by a layer 204 of debris or dirt deposited on the second window face 224b. The layer 204 may not be a continuous layer over the first window face 224a or the second window face 224b. The layer 204 may be of varying thickness.

As illustrated in FIG.1 and FIG.2, the protective assembly 102 further comprises an optical sensor 126 configured to measure an optical parameter of the portion of light 200 scattered at the protective window 124. According to an embodiment, the optical sensor 126 comprises a photodiode, a phototransistor, a photoresistor, an array of photodiodes, phototransistors, or phototransistors, or a camera. According to an embodiment, the optical sensor 126 is sensitive to the first wavelength, the second wavelength or to both wavelengths. According to an embodiment, the optical parameter is an optical power, an optical intensity, or an optical polarization.

An active area 206 of the optical sensor 126 is an area that can convert photons to an electrical signal, such as an electrical current. According to an embodiment, the electrical current is proportional to the number of photons, or optical power, that are incident on the active area 206 of the optical sensor 126. The electrical current can then be converted to a voltage using appropriate circuitry, such as a transimpedance amplifier. Optical intensity is defined as the power per unit area. Therefore, if the area of the active area is known, then the optical intensity can be determined from the optical power. According to an embodiment, the optical sensor 126 comprises an optical lens (not shown) configured to direct light on the active area 206 of the optical sensor 126.

According to an embodiment, light from the aiming beam 112 or therapeutic beam 104 that is reflected at the first window face 124a and the second window face 124b is at least partially polarized following scattering. According to an embodiment, the optical sensor 126 is a polarization analyzer configured to measure the optical polarization of the scattered light.

According to an embodiment, the optical sensor 126 is not disposed on the first optical path or the second optical path and is disposed at a position relative to the protective window 124 so as to measure the optical parameter of the light from the therapeutic laser beam 104 and/or the aiming beam 112 scattered at the protective window 124.

Referring to FIG.2, it is clear that scattering may occur in many directions depending on the nature of the defect or contamination of the protective window 124. As a result, the optical sensor 126 may be placed in various positions relative to the protective window 124 so long as at least some of scattered light is incident on the active area 206 of the optical sensor 126. An optical axis 306 of the optical sensor 126 is an axis that passes through the center of the active area 206 perpendicular to a plane comprising the active area 206. An acceptance angle of the optical sensor 126 is the maximum angle with respect to the optical axis 306 of the optical sensor 126 at which light will be incident on the optical sensor 126. The acceptance angle defines a cone of acceptance 208 (also known as the angular optical field). According to an embodiment, the optical sensor 126 is placed relative to the protective window 124 so that the cone of acceptance 208 of the optical sensor 126 encompasses at least part of the protective window 124, wherein the cone of acceptance 208 extends at least 1mm from the first window face 124a of the protective window 124 in the direction of the optical free space area 114 and/or extends at least 1mm from the second window face 124b of the protective window (124) in a direction of the treatment area.

Referring to FIG.2, according to an embodiment, the optical sensor126 is positioned relative to the protective window 124 so as to measure scattering in a direction substantially parallel to the first window face 124a and the second window face 124b.

According to an embodiment, the optical sensor 126 is positioned relative to the protective window 124 so as to measure the optical parameter of light scattered in a direction forming an angle less than 20° with a plane of the first window face 124a and/or forming an angle less than 20° with a plane of the second window face 124b.

It will be clear to a person of ordinary skill in the art that the positioning of the optical sensor 126 may depend on a sensitivity of the optical sensor 126, an active area 206 of the optical sensor 126 and other parameters of the therapeutic laser system 100, such as a strength or power of the therapeutic laser beam 104 or the aiming beam 112 at the protective window 124. For example only, placing the optical sensor 126 too close to where the therapeutic laser beam 104 and the aiming beam 112 substantially pass through the protective window 124 may result in a saturation of the optical sensor 126. For example only, placing the optical sensor 126 too far from where the therapeutic laser beam 104 and the aiming beam 112 substantially pass through the protective window 124 may result in a low level of light that is difficult to differentiate from a noise level.

According to an embodiment, the optical sensor 126 is configured to detect the second wavelength and not the first wavelength. This can be achieved by selecting an optical sensor with a detection range comprising the second wavelength and not the first wavelength. Alternatively, an optical filter (not shown) may be placed in front of the optical sensor 126, where the optical filter is configured to filter (or substantially attenuate) the first wavelength. When the second wavelength is in the visible range of the wavelengths of light, photodiodes for use in the visible range generally have a lower price than those in the infrared range.

According to an embodiment, a power of the aiming beam 112 is lower than a power of the therapeutic beam 104. The power of the aiming beam 112 is such that it can be used with a low risk or with no risk of damaging the window or risk of causing fire. For example, if there is dirt on the protective window 124, due to the higher power, the therapeutic laser beam 104 will burn the dirt and further damage the protective window 124. On the other hand, due to its lower power compared to the therapeutic laser beam 104, there is a low risk or no risk of the aiming beam 112 burning the dirt on the protective window. As a result, the aiming beam 104 can be used to monitor the protective window 124 even after an emergency stop of the laser.

The protective assembly 102 further comprises a control unit 128. The optical sensor 126 may be communicatively coupled to the control unit 128. The control unit 128 may be electrically coupled to the optical sensor 126. Alternatively, the control unit 128 may be remote from the optical sensor, such as within a remote computer, or on a cloud computing system, and configured to receive the measured optical parameter from the optical sensor 126. The control unit 128 comprises a computer processor and is configured to determine if the optical parameter measured by the optical sensor 126 is within a predetermined range. According to an embodiment, if the measured optical parameter is within a predetermined range then the protective window 124 is identified as being damaged or uncontaminated. The predetermined range may represent values for the optical parameter for which the protective window 124 can be considered to be damaged or contaminated.

According to an embodiment, the control unit 128 is configured to compare the measured optical parameter to a baseline measurement, where the baseline measurement was obtained for the protective window 124 as part of a calibration or quality checking procedure. The baseline measurement represents the scattering of the therapeutic beam and/or aiming beam for the protective window 124 which has not been damaged. In an embodiment, the control unit 128 is configured to determine if the measured optical parameter is within a range of values that does not include the baseline measurement, for which the protective window 124 can be identified as damaged or contaminated.

According to an embodiment, the control unit 128 is configured to compare the measured optical parameter to a limit value, where the limit value indicates a value of the optical parameter which is associated with a defect or contamination of the protective window 124. In an embodiment, the control unit 128 is configured to determine if the measured optical parameter is within a predetermined range with respect to the limit value. In an embodiment, the predetermined range may comprise the limit value, and if the optical parameter is within the predetermined range the protective window 124 may be identified as being damaged or contaminated.

According to an embodiment, if the control unit 128 determines that the optical parameter measured by the optical sensor 126 is within a predetermined range it may indicate to a user that the protective window 124 should be inspected. For example, the control unit 128 may cause a message to be displayed to a user or an alarm to be sounded. According to an embodiment, the control unit 128 may further be configured to inhibit operation of the therapeutic laser beam 104 when the optical parameter measured by the optical sensor 126 is within the predetermined range.

According to an embodiment, the therapeutic laser system 100 may further include an optical fiber connector (not shown) configured to mechanically couple to the proximal end 106a of the optical fiber 106, so as to hold the proximal end 106a of the optical fiber 106 in place along the first optical path and the second optical path. An example of such a connector includes subminiature version A (SMA), ferrule connector (FC), straight tip (ST), and lucent connector (LC). In such an embodiment, the connector is positioned along the first optical path and along the second optical path between the protective window 124 and the proximal end 106a of the optical fiber 106 so that the therapeutic laser beam 104 and the aiming beam 112 can be directed to the proximal end 106a of the optical fiber 106.

According to an embodiment, the therapeutic laser system 100 may be configured to direct the therapeutic laser beam 104 to the treatment site without the optical fiber 106. In this case, the protective window 124 may be placed in contact with or proximal to the treatment area so that the therapeutic laser beam 104 and the aiming beam 112 are at least partially transmitted through the protective window 124 to irradiate the treatment area.

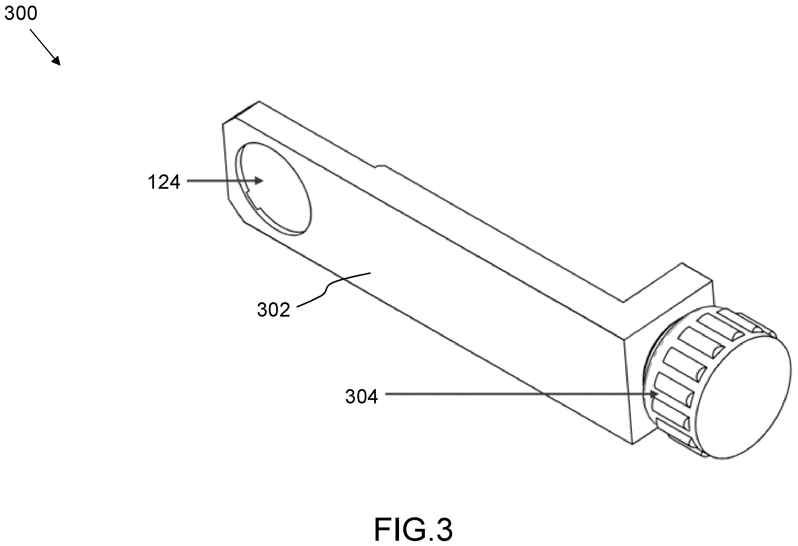

FIG. 3 illustrates a removable mounting 300 configured to hold or support the protective window 124 according to an embodiment. The removable mounting 300 comprises a window support member 302 configured to hold or support the protective window 124 and an attachment member 304. The removable mount 300 ensures that the protective window 124 is inserted into the correct place relative to the optical free space area 114 within the therapeutic laser system 100. The window support member 302 may be made of metal, plastic or any material suitable for supporting the protective window 124. According to an embodiment, the window support member 302 forms a frame substantially around a periphery of the protective window 124. The window support member 302 does not obstruct or cover the first window face 124a or the second window face 124b of the protective window 124.

The window support member 302 may also support or hold the optical sensor 126 (not shown in FIG. 3) at a fixed position relative to the protective window. Alternatively, the optical sensor 126 may not be supported by the window support member 302 and may be fixed separately within the therapeutic laser system 100 so that when the removable mounting 300 with the protective window 124 is inserted into the therapeutic laser system, the optical sensor 126 is at a predetermined fixed position relative to the protective window 124.

The attachment member 304 is mechanically coupled to the window support member 302 and enables the removable mounting 300 to be releasably attached to the therapeutic laser system 100, such as to a housing of the optical free space area 114. The attachment member 304 may be a manual screw, as illustrated in FIG. 3. Alternatively, the attachment member 304 may be a clip, magnet or other attachment means.

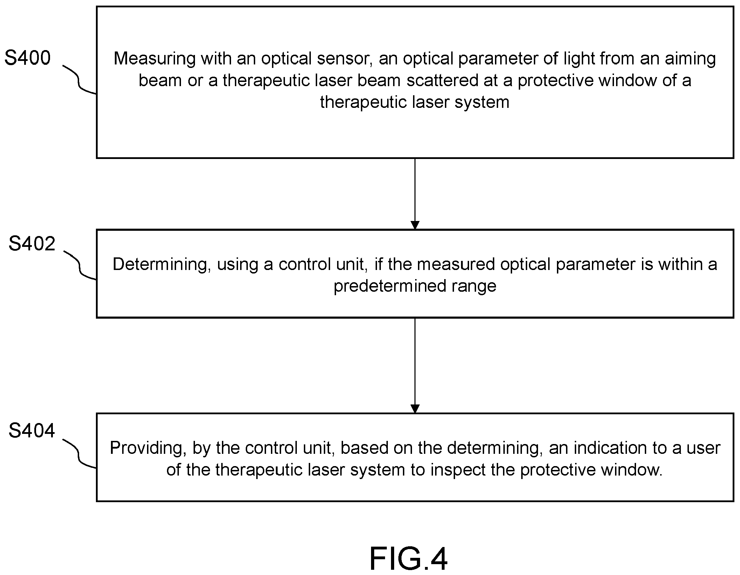

FIG. 4 illustrates the method steps to monitor the protective window 124 used in the therapeutic laser system 100 according to an embodiment. At S400, the optical sensor 126 measures at least one optical parameter of light from the aiming beam 112 or the therapeutic laser beam 104 scattered at the protective window 124 of the therapeutic laser system 100. At S402, the control unit 128 determines if the measured optical parameter is within a predetermined range. Optionally, at S404, the control unit 128 provides, based on the determining, an indication to a user of the therapeutic laser system 100 to inspect the protective window 124.

While the invention has been described with respect to a limited number of embodiments, those skilled in the art, having benefit of this disclosure, will appreciate that other embodiments can be devised which do not depart from the spirit of the invention as disclosed herein. Accordingly, the scope of the invention should be limited only by the attached claims.

### REFERENCES

[1] US20020186366A1 "Apparatus and method of monitoring and controlling power output of a laser system".
[2] EP0212786A1 "Laser/optical fiber safety apparatus and method".
[3] EP1265322A2 "Apparatus and method of directing a laser beam to a thermally managed beam dump in a laser system".
[4] https://www.ems-urology.com/products/swiss-laserclast
[5] IEC 60601-2-22:2019 RLV ^{©} IEC 2019., paragraph 201.3.202

## Claims

1. A protective assembly (102) for use in a therapeutic laser system (100), the therapeutic laser system (100) configured to direct a therapeutic laser beam (104) to a treatment area, the therapeutic laser system (100) comprising a laser unit and an optical free space area (114) disposed between the laser unit and the treatment area, the laser unit being configured to output the therapeutic laser beam (104) having a first optical path through said optical free space area to the treatment area and to output an aiming beam (112) having a second optical path through said optical free space area to the treatment area, the protective assembly (102) comprising:
a protective window (124) configured to be disposed between the optical free space area (114) and the treatment area so as to intersect the first optical path and the second optical path, the protective window (124) at least partially transparent to the therapeutic laser beam (104) and to the aiming beam (112);
an optical sensor (126) configured to measure an optical parameter of light from the aiming beam (112) or the therapeutic laser beam (104) scattered at the protective window (124); and
a control unit (128) configured to determine if the measured optical parameter is within a predetermined range.

2. The protective assembly (102) according to claim 1, wherein the protective window (124) is at least partially transparent in a range of about 280 nm to about 100 µm.

3. The protective assembly (102) according to claim 1 or claim 2, wherein the control unit (128) is further configured to provide an indication to a user of the therapeutic laser system (100) to inspect the protective window (124).

4. The protective assembly (102) according to any one of claims 1 to 3, wherein the optical sensor (126) is positioned relative to the protective window (124) so that a cone of acceptance (208) of the optical sensor (126) encompasses at least part of the protective window (124), wherein the cone of acceptance (208) extends at least 1mm from a first window face (124a) of the protective window (124) in the direction of the optical free space area (114) and/or extends at least 1mm from a second window face (124b) of the protective window (124) in a direction of the treatment area, wherein the cone of acceptance (208) defines a maximum angle with respect to an optical axis of the optical sensor (126) at which light will be incident on an active area of the optical sensor (126).

5. The protective assembly (102) according to any one of claims 1 to 3 wherein the optical sensor (126) is positioned relative to the protective window (124) so as to measure the optical parameter of the light scattered in a direction forming an angle less than 20° with a plane of a first window face (124a) of the protective window (124) and/or forming an angle less than 20° with a plane of a second window face (124b) of the protective window (124).

6. The protective assembly (102) of any one of claims 1 to 5, wherein the protective window (124) comprises an anti-reflection coating.

7. The protective assembly (102) according to any one of claims 1 to 6, wherein the optical parameter is an optical intensity, a polarization or an optical power.

8. The protective assembly (102) according to any one of claims 1 to 7, further comprising a removable mounting (300), the removable mounting (300) comprising:
a window support member (302) configured to support the protective window (124); and
an attachment member (304) mechanically coupled to the window support member (302) and configured to releasably attach the protective assembly (102) to a housing of the therapeutic laser system (100).

9. A therapeutic laser system (100) configured to direct a therapeutic laser beam (104) to a treatment area, the therapeutic laser system (100) comprising:
a laser unit configured to output the therapeutic laser beam (104) having a first optical path through an optical free space area to the treatment area and to output an aiming beam (112) having a second optical path through the optical free space area to the treatment area;
the optical free space area (114) disposed between the laser unit and the treatment area; and
a protective assembly (102) according to any one of claims 1 to 8.

10. The therapeutic laser system (100) according to claim 9, wherein the laser unit comprises a first light source (108) configured to output the therapeutic laser beam (104), the first light source (108) being one of a Thulium:YAG laser, a Holium:YAG laser, a KTP (Potassium titanyl phosphate):YAG laser, Nd (Neodymium):YAG laser, a carbon dioxide (CO₂) laser or a diode laser.

11. The therapeutic laser system (100) according to claim 9 or claim 10, wherein the laser unit comprises a second light source (110) configured to output the aiming beam (112), the second light source (100) being one of a laser diode, a laser or a light emitting diode configured to emit light in the range of 280 nm to 700 nm.

12. The therapeutic laser system (100) according to any one of claims 9 to 11, wherein the optical free space area (114) comprises a lens (116) configured to direct the therapeutic laser beam (104) and the aiming beam (112) toward a proximal end (106a) of an optical fiber (106), the optical fiber (106) disposed between the protective window and the treatment area, and wherein the protective window (124) of the protective assembly (102) is disposed between the lens (116) and the proximal end (106a) of the optical fiber (106).

13. The therapeutic laser system (100) according to claim 12 further comprising:
a fiber coupler configured to couple to the optical fiber (106); and, optionally,
the optical fiber (106).

14. A method of monitoring a protective window (124) of a therapeutic laser system (100) configured to direct a therapeutic laser beam (104) to a treatment area, the therapeutic laser system (100) comprising a laser unit configured to output the therapeutic laser beam (104) having a first optical path through an optical free space area to the treatment area and to output an aiming beam (112) having a second optical path through the optical free space area to the treatment area, the laser system (100) further comprising the optical free space area (114) disposed between the laser unit and the treatment area, and a protective window (124) disposed between the optical free space area (114) and treatment area so as to intersect the first optical path and the second optical path, the protective window (124) at least partially transparent to the therapeutic laser beam (104) and the aiming beam (112), the method comprising:
measuring with an optical sensor (126) an optical parameter of light from the aiming beam (112) or the therapeutic laser beam (104) scattered at the protective window (124); and
determining, using a control unit (128), if the measured optical parameter is within a predetermined range.

15. The method of claim 14 further comprising:
providing, by the control unit (128), based on the determining, an indication to a user of the therapeutic laser system (100) to inspect the protective window (124).
